# EUROPEAN PATENT SPECIFICATION

(11) **EP 3 923 791 B1**
(45) Date of publication and mention of the grant of the patent: **11.11.2026**
(21) Application number: 20705812.4
(22) Date of filing: 03.02.2020
(51) Int. Cl.: A61B 5/00, A61B 5/282, A61B 5/339, A61B 5/35, A61B 5/363, A61B 5/364, A61B 5/346

(54) **ELECTROCARDIOGRAM WAVEFORM ANALYSIS APPARATUS**
ELEKTROKARDIOGRAMMWELLENFORMFORMANALYSEVORRICHTUNG
APPAREIL D'ANALYSE DE FORME D'ONDE D'ÉLECTROCARDIOGRAMME

(30) Priority: 14.02.2019 JP 2019024876
(43) Date of publication of application: 22.12.2021
(73) Proprietor: Nihon Kohden Corporation, Shinjuku-ku Tokyo 161-8560 (JP)
(72) Inventor: NATSUI, Tomoyoshi, Tokorozawa-shi, Saitama 359-0037 (JP)
(74) Representative: Grünecker Patent- und Rechtsanwälte PartG mbB
(86) International application number: PCT/JP2020/003965
(87) International publication number: WO 2020/166413

(56) References cited:
- US-A1- 2017 143 976
- JIAPU PAN ET AL: "A Real-Time QRS Detection Algorithm", IEEE TRANSACTIONS ON BIOMEDICAL ENGINEERING, vol. BME-32, no. 3, 1 March 1985 (1985-03-01), pages 230 - 236, XP055129317, ISSN: 0018-9294, DOI: 10.1109/TBME.1985.325532
- MAROUF MOHAMED ET AL: "Adaptive EMG noise reduction in ECG signals using noise level approximation", PROCEEDINGS OF SPIE; [PROCEEDINGS OF SPIE ISSN 0277-786X VOLUME 10524], SPIE, US, vol. 10613, 19 December 2017 (2017-12-19), pages 106130E - 106130E, XP060098332, ISBN: 978-1-5106-1533-5, DOI: 10.1117/12.2299841
- VADREVU SIMHADRI ET AL: "A Robust Pulse Onset and Peak Detection Method for Automated PPG Signal Analysis System", IEEE TRANSACTIONS ON INSTRUMENTATION AND MEASUREMENT, IEEE SERVICE CENTER, PISCATAWAY, NJ, US, vol. 68, no. 3, 13 August 2018 (2018-08-13), pages 807 - 817, XP011709673, ISSN: 0018-9456, [retrieved on 20190207], DOI: 10.1109/TIM.2018.2857878

## Description

### Technical Field

The presently disclosed subject matter relates to an electrocardiogram waveform analysis apparatus in which an erroneous determination of beats can be eliminated.

### Background Art

Conventionally, an electrocardiogram waveform collected from a subject is signal processed, and then it is determined whether beats contained in the electrocardiogram waveform are extrasystolic beats or beats other than extrasystolic beats.

Usually, an electrocardiogram waveform contains noises due to body motion of the subject such as the muscle activity. When the noises are not successfully eliminated, an erroneous determination of beats is caused.

Patent Literatures 1 and 2 below disclose a technique for eliminating such noises. In the techniques disclosed in Patent Literatures 1 and 2, blurring of an electrocardiogram signal due to body motion of the subject, i.e., motion artifacts are reduced. In the techniques, specifically, a residual signal is produced from an electrocardiogram waveform, and the residual signal is subtracted from the electrocardiogram waveform, thereby reducing motion artifacts.

### Citation List

### Patent Literature

PTL 1: JP-T-2016-517712
PTL 2: Japanese Patent No. 6,235,608

The article "A Real-Time QRS Detection Algorithm" (Pan et al., IEEE Transactions on Biomedical Engineering), Vol. BME-32, No. 3 (1985)) discloses a real-time algorithm for detection of the QRS complexes in ECG signal based upon digital analyses of slope, amplitude and width.

US 2017/0143976 A1 relates to a computer-implemented method for detection of onset of depolarization on electrocardiogram signals. The method, inter alia, includes determining a baseline rhythm, including an atrial marker and a ventricular marker, using a plurality of body-surface electrodes, defining a pre-specified window as being between the atrial marker and the ventricular marker, and applying a low pass filter to a signal within the window.

### Summary of Invention

### Technical Problem

Even when the techniques disclosed in Patent Literatures 1 and 2 are applied to an electrocardiogram waveform collected from the subject, however, it is difficult to eliminate an erroneous determination of beats. For example, an erroneous determination of whether beats are extrasystolic beats or those other than extrasystolic beats is difficult to be eliminated. Since extrasystolic beats are most frequent as a cause of arrhythmia, an erroneous determination of beats degrades the reliability of diagnosis of arrhythmia. Moreover, a phenomenon in which a portion that is not a beat is erroneously detected as a beat may occur.

Therefore, it is an object of the presently disclosed subject matter to provide an electrocardiogram waveform analysis apparatus in which an erroneous determination of beats can be eliminated. This object is solved by the subject-matter of the appended claims.

### Solution to Problem

The electrocardiogram waveform analysis apparatus according to claim 1 includes a waveform input section, an application range setting section, a filtering section, a beat detector, and an output section.

An electrocardiogram waveform is input to the waveform input section. The application range setting section is configured to set portions of the electrocardiogram waveform that are other than the QRS wave of the electrocardiogram waveform as an application range of a filter in the input electrocardiogram waveform to define the application range of the filter that excludes the QRS wave of the input electrocardiogram waveform and comprises only one or more portions located outside the QRS wave. The filtering section is configured to apply a filtering process only to the application range of the filter while leaving the QRS wave, the filtering process being configured to reduce noise contained in the filter application range. The beat detector is configured to detect beats from the electrocardiogram waveform that has undergone the filtering process. The output section is configured to perform an outputting process based on the detected beats.

According to the electrocardiogram waveform analysis apparatus of the presently disclosed subject matter, an erroneous determination of beats can be eliminated.

### Brief Description of Drawings

[fig. 1]FIG. 1 is a block diagram of an electrocardiogram waveform analysis apparatus of an embodiment.
[fig.2]FIG. 2 is an operation flowchart of the electrocardiogram waveform analysis apparatus of the embodiment.
[fig.3]FIG. 3 illustrates an example of an electrocardiogram waveform that is input to a waveform input section.
[fig.4]FIG. 4 illustrates a filter application range that is set by an application range setting section.
[fig.5]FIG. 5 illustrates an electrocardiogram waveform that has been filtered by a filtering section.
[fig.6]FIG. 6 illustrates beats that are detected by a beat detector, and kinds of beats that are analyzed by a beat analyzer.
[fig.7]FIG. 7 illustrates a mode of outputting an electrocardiogram waveform and kinds of beats that are output from an output section.
[fig.8A]FIG. 8A illustrates electrocardiogram waveforms of a plurality of channels that are input to the waveform input section.
[fig.8B]FIG. 8B illustrates the electrocardiogram waveforms of the plurality of channels that have been filtered.
[fig.9A]FIG. 9A illustrates a mode of outputting electrocardiogram waveforms to which the presently disclosed subject matter has not yet been applied, and kinds of beats.
[fig.9B]FIG. 9B illustrates a mode of outputting the electrocardiogram waveforms to which the presently disclosed subject matter has been applied, and the kinds of beats.

### Description of Embodiments

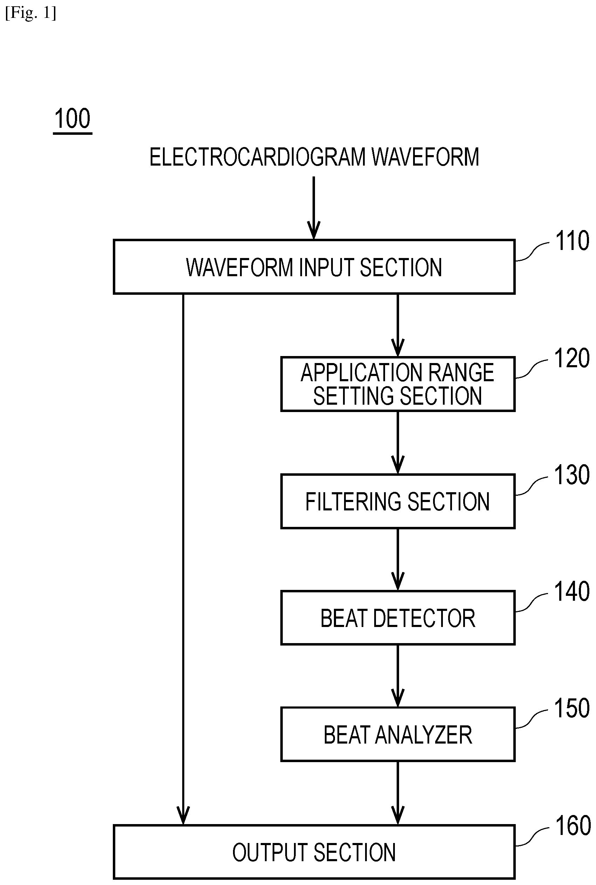

Hereinafter, an embodiment of the electrocardiogram waveform analysis apparatus of the presently disclosed subject matter will be described in detail with reference to the drawings. FIG. 1 is a block diagram of an electrocardiogram waveform analysis apparatus of the embodiment.

### <Configuration of electrocardiogram waveform analysis apparatus>

The electrocardiogram waveform analysis apparatus 100 may include a waveform input section 110, an application range setting section 120, a filtering section 130, a beat detector 140, a beat analyzer 150, and an output section 160. The electrocardiogram waveform analysis apparatus 100 is a medical apparatus for measuring an electrocardiogram, such as a patient monitor, a defibrillator, a 12-lead electrocardiograph, or a Holter electrocardiograph. Alternatively, the electrocardiogram waveform analysis apparatus 100 may be an arbitrary analysis apparatus that acquires waveform information from a patient monitor, a Holter electrocardiograph, or the like, and that displays analysis results.

The waveform input section 110 acquires electrocardiogram signals of a subject from sensors such as electrocardiogram electrodes that are not illustrated. Then, an electrocardiogram waveform over a predetermined time period is input from the waveform input section 110 to the application range setting section 120 and the output section 160. In the case where the electrocardiogram waveform analysis apparatus is a patient monitor, a defibrillator, or a 12-lead electrocardiograph, for example, an electrocardiogram waveform that is measured for a predetermined time period (for example, several seconds) is suppled in real time from the waveform input section 110 in order to determine arrhythmia. In the case where the apparatus is a Holter electrocardiograph, an electrocardiogram waveform that is measured for a predetermined time period (for example, 24 hours) is suppled in real time from the waveform input section 110 in order to carefully examine the cause of arrhythmia or angina. From the waveform input section 110, in this way, an electrocardiogram waveform is input in real time, or a recorded electrocardiogram waveform is input.

The application range setting section 120 sets a filter application range in the electrocardiogram waveform that is input from the waveform input section 110. While, in the electrocardiogram waveform that is input from the waveform input section 110, setting an amplitude that is within the predetermined time period, and that satisfies a predetermined standard, as a reference, for example, the application range setting section 120 sets waveform portions having an amplitude that is equal to or smaller than a predetermined ratio of the amplitude set as the reference, as the application range. In short, an electrocardiogram waveform over a predetermined time period (for example, 10 seconds) is divided into portions having a larger amplitude, and those having a smaller amplitude, and the portions having a smaller amplitude are set as the filter application range. Therefore, according to the invention, the application range setting section 120 can set portions of an electrocardiogram waveform that are other than the QRS wave, as the filter application range, and prevent the waveform of the QRS wave from blurring.

The filtering section 130 applies a filtering process to the filter application range that is set by the application range setting section 120. In the filtering section 130, for example, an analog filter such as an RC filter in which a resistor and a capacitor are used, or an LC filter in which a coil and a capacitor are used, or a digital filer in which a calculation technique such as the moving average method is used, as the filter. In the case where an analog filter is used, a calculation of the filtering process is not necessary, and therefore the filtering process can be accelerated. In the case where a digital filter is used, an adjustment of filtering, such as the frequency range of noises to be eliminated and the degree of elimination can be easily performed, and therefore the filtering process is optimized.

The beat detector 140 detects beats from the electrocardiogram waveform that has been filtered by the filtering section 130. The detection of beats is performed on the electrocardiogram waveform that has been filtered, and portions having an amplitude that is equal to or higher than a predetermined amplitude are detected as beats.

The beat analyzer 150 analyzes the beats that are detected by the beat detector 140, and classifies the kinds of beats. In the beat analyzer 150, for example, the kinds of beats are classified by using pattern matching. The beats are classified into a plurality of kinds including the normal beat (N) and the ventricular extrasystolic beat (V). When beats are analyzed by the beat analyzer 150 by using pattern matching, it is possible to many kinds of beats, and it is further possible to eliminate erroneous determination of beats.

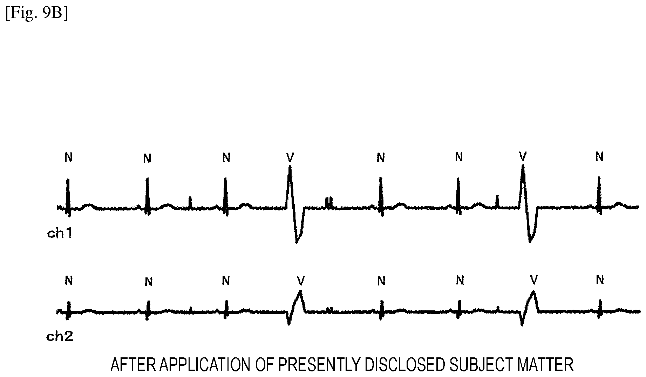

The output section 160 is a display device in which a liquid crystal or an organic El is used, or a printer, and performs an outputting operation (a display or a print output) based on the beats that are detected by the beat detector 140, and analyzed by the beat analyzer 150. Specifically, the output section 160 outputs, together with the electrocardiogram waveform that is input from the waveform input section 110, the kinds of beats that are classified by the beat analyzer 150. The classified kinds of beats are output in tune with the waveform portions of the beats of the electrocardiogram waveform that is input from the waveform input section 110 (see the display mode of FIG. 9B). Since the output section 160 is a display device or a printer, a display or a print output can be performed based on the beats that are analyzed by the beat analyzer 150. Since the output section 160 further outputs the kinds of beats together with the input electrocardiogram waveform, it is possible to check the electrocardiogram waveform and the kinds of beats in comparison with each other. Moreover, the kinds of beats are output in tune with the waveform portions of the kinds of beats of the input electrocardiogram waveform, and therefore the correspondence relationship among the waveform portions and the kinds of beats can be known at a glance. Alternatively, the output section 160 may be a speaker that outputs a sound in accordance with beats. Moreover, the output section 160 may have a function of producing display data (for example, HTML data) that can be displayed in an external apparatus such as a smart phone or a tablet terminal, and transmitting the display data to the external apparatus (the smart phone or the tablet terminal), and an outputting operation that is based on the beats may be performed by the external apparatus.

### <Operation of electrocardiogram waveform analysis apparatus>

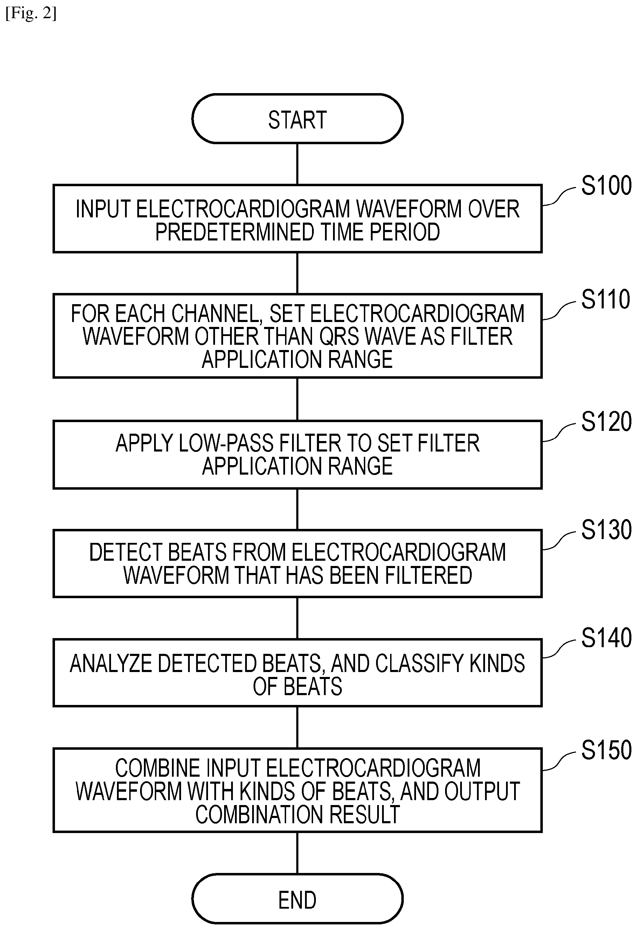

The configuration of the electrocardiogram waveform analysis apparatus 100, and schematic operations of the components have been described above. Next, the operation of the electrocardiogram waveform analysis apparatus 100 will be described in detail with reference to the operation flowchart of FIG. 2. FIG. 2 is an operation flowchart of the electrocardiogram waveform analysis apparatus of the embodiment.

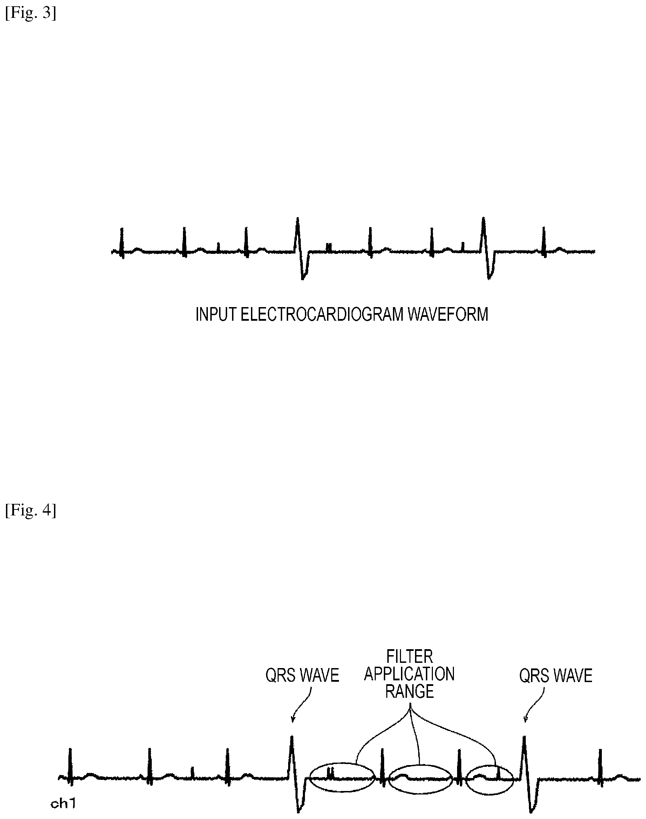

An electrocardiogram waveform over a predetermined time period is input from the waveform input section 110 (S100). FIG. 3 illustrates an example of the electrocardiogram waveform that is input to the waveform input section 110. In the case of an electrocardiogram waveform that is recorded by a Holter electrocardiograph, for example, an electrocardiogram waveform extending for 24 hours as illustrated in FIG. 3 is input from the waveform input section 110. Although FIG. 3 illustrates an electrocardiogram waveform of one channel in order to facilitate the understanding of the presently disclosed subject matter, electrocardiogram waveforms of a plurality of channels are actually input from the waveform input section 110.

For each of the channels, the application range setting section 120 sets portions of the electrocardiogram waveform other than the QRS wave as the filter application range (S110). FIG. 4 illustrates the filter application range that is set by the application range setting section 120. While, in the electrocardiogram waveform illustrated in FIG. 3, setting an amplitude (e.g., the maximum amplitude of the QRS wave) that is within the predetermined time period (e.g., 10 seconds), and that satisfies a predetermined standard, as a reference, for example, the application range setting section 120 sets a waveform portion having an amplitude that is equal to or smaller than a predetermined ratio (e.g., 20% or smaller) of the amplitude set as the reference, as the application range. That is, an electrocardiogram waveform is divided into a portion of the QRS wave and having a larger amplitude, and that other than the QRS wave and having a smaller amplitude, and the portion having a smaller amplitude is set as the filter application range. In a predetermined time period of the electrocardiogram waveform illustrated in FIG. 3, therefore, the filter application range that is set by the application range setting section 120 is the portions that are enclosed respectively by the ellipses in FIG. 4, and that have a smaller amplitude. An epsilon-filter may be used in the setting of the filter application range. This is because an epsilon -filter is a type of linear filter, and has characteristics in which a filtering process is not applied to a portion having a larger amplitude, but applied to that having a smaller amplitude.

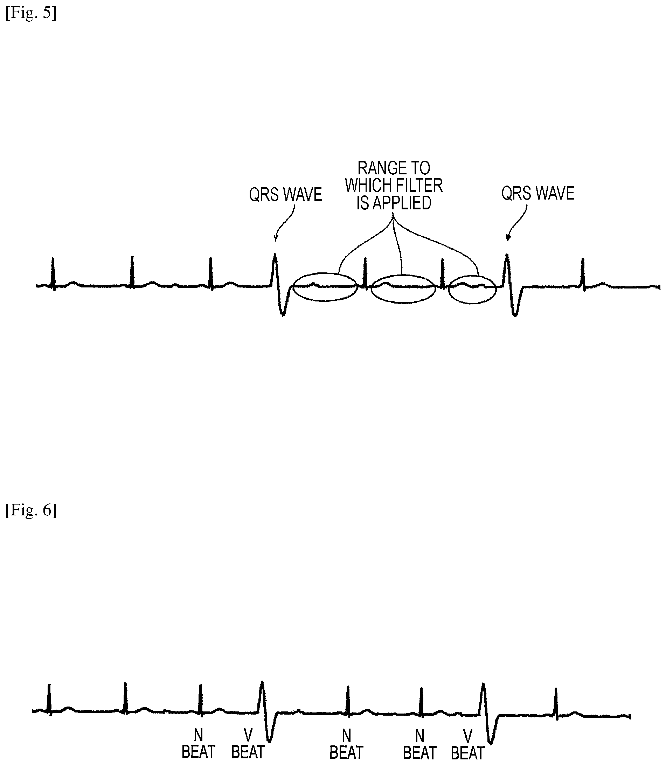

The filtering section 130 applies a low-pass filter to the filter application range that is set by the application range setting section 120 (S120). FIG. 5 illustrates an electrocardiogram waveform that has been filtered by the filtering section 130. When a low-pass filter is applied to the electrocardiogram waveform in the filter application range, as illustrated in FIG. 5, the electrocardiogram waveform in the application range is smoothed as compared with FIG. 4. As a result of smoothing of the electrocardiogram waveform, noises are eliminated, and the shape of the QRS wave becomes conspicuous. The shape of the QRS wave is important in determination of the kinds of beats. When the shape of the QRS wave is conspicuous, therefore, it is possible to eliminate erroneous determination of the kinds of beats.

The beat detector 140 detects beats from the electrocardiogram waveform that has been filtered (S130). FIG. 6 illustrates beats that are detected by the beat detector 140, and the kinds of beats that are analyzed by the beat analyzer 150. The beat detector 140 detects portions that are in the electrocardiogram waveform illustrated in FIG. 5, and that have an amplitude which is equal to or higher that the predetermined amplitude, as a beat. The detected beats are four electrocardiogram waveforms portions illustrated in FIG. 6.

The beat analyzer 150 analyzes the beats detected by the beat detector 140, and classifies the kinds of beats (S140). In the beat analyzer 150, patterns of plural kinds of electrocardiogram waveforms, including the normal beat (N) and the ventricular extrasystolic beat (V) are registered. The beat analyzer 150 collates the registered patterns of electrocardiogram waveforms with the patterns of the electrocardiogram waveforms of the beats detected by the beat detector 140, by using pattern matching, and classifies the kinds of beats. For example, the kinds of beats are classified into at least the normal beat (N) and the ventricular extrasystolic beat (V). As illustrated in FIG. 6, an electrocardiogram waveform portion having a smaller amplitude is classified into the N beat, and that having a larger amplitude is classified into the V beat.

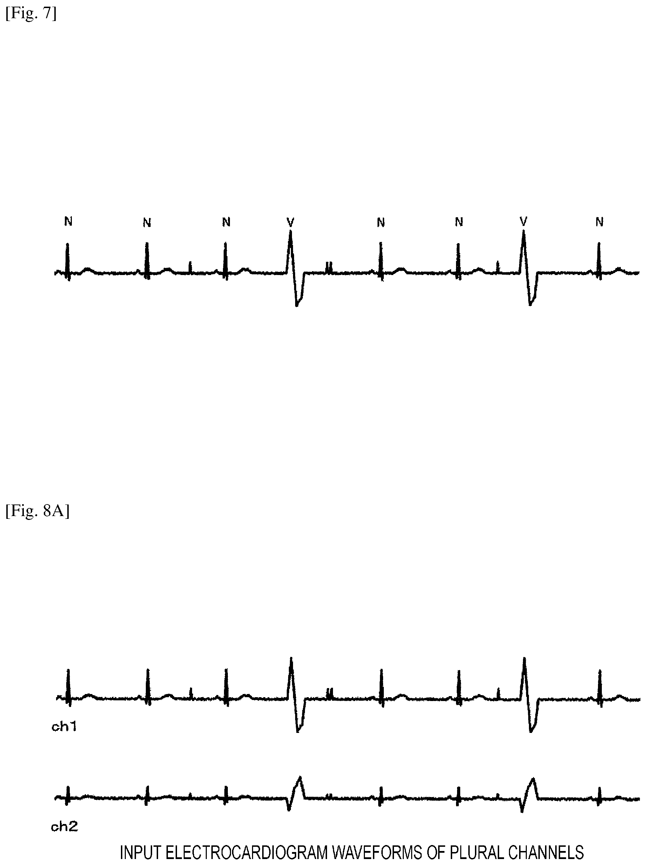

The output section 160 combines the input electrocardiogram waveform with the kinds of beats, and outputs the combination result (S150). FIG. 7 illustrates a mode of outputting the electrocardiogram waveform and kinds of beats that are output from the output section 160. In the case where the output section 160 is a display device in which a liquid crystal or an organic El is used, the electrocardiogram waveform input from the waveform input section 110 (the raw waveform that has not yet undergone the process of the operation flowchart of FIG. 2) is combined with the kinds of beats that are determined by the beat analyzer 150, and the combination result is displayed on the display device. While the letter "N" is disposed above the normal beads of the raw waveform, and the letter "V" is disposed above the ventricular extrasystolic beats of the raw waveform, namely, the combination result is displayed on the display device. In place of the mode of outputting the combination of the suppled electrocardiogram waveform and the kinds of beats, another outputting mode may be employed where information indicating that beats are detected may be combined with the input electrocardiogram waveform (for example, a mark such as a graphic symbol or a symbol is displayed above each of the beats), and the combination result is displayed. In the case where the output section 160 is a printer, an image that is same as or similar to FIG. 7 is printed out.

As described above, the electrocardiogram waveform analysis apparatus 100 of the embodiment applies a filtering process to, in the input electrocardiogram waveform, the electrocardiogram waveform portions other than the QRS wave to eliminate noises, and determines the kinds of beats from the electrocardiogram waveform from which noises have been eliminated. In a portion of an electrocardiogram waveform in which the amplitude is lower than that of the QRS wave, and which is other than the QRS wave, noises that are due to body motion of the subject or the like, and that are contained in the electrocardiogram waveform cause the determination of the kinds of beats to be erroneously performed. When, as in the electrocardiogram waveform analysis apparatus 100 of the embodiment, the electrocardiogram waveform portions other than the QRS wave are filtered while leaving the QRS wave, therefore, an erroneous determination of beats (a portion that is not a beat is determined as a beat, a beat is determined to be not a beat, or the kind of a beat is erroneously determined) is eliminated, and the reliability of a diagnosis of arrhythmia is improved.

### <Comparison between before and after application of presently disclosed subject matter>

Next, results of comparisons of cases in which a diagnosis of arrhythmia was performed with or without using the electrocardiogram waveform analysis apparatus 100 of the embodiment will be described.

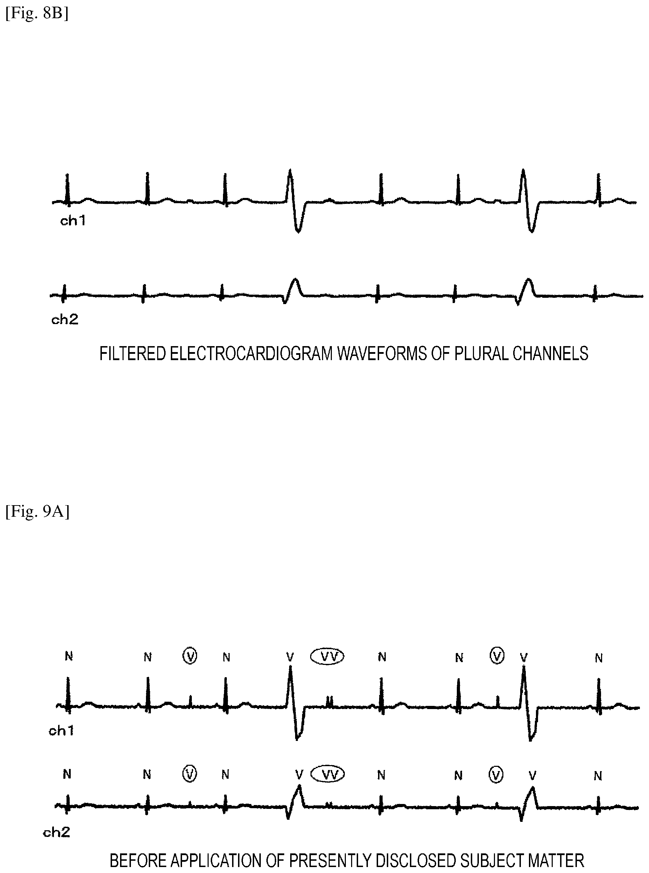

FIG. 8A illustrates electrocardiogram waveforms of a plurality of channels that are input to the waveform input section 110, FIG. 8B illustrates the electrocardiogram waveforms of the plurality of channels that have been filtered, FIG. 9A illustrates a mode of outputting electrocardiogram waveforms to which the presently disclosed subject matter has not yet been applied, and kinds of beats, and FIG. 9B illustrates a mode of outputting the electrocardiogram waveforms to which the presently disclosed subject matter has been applied, and the kinds of beats.

Before description of comparison between before and after application of the presently disclosed subject matter, an edition work that is performed in an editing center will be described.

In the case of a Holter electrocardiograph, electrocardiogram waveforms of a subject for 24 hours are recorded. The recorded electrocardiogram waveform data for 24 hours are delivered to an editing center, and then analyzed by an electrocardiogram waveform analysis apparatus, and an edition is performed in order to diagnose arrhythmia.

In the editing center, the editor visually checks, together with the electrocardiogram waveform, results of determinations of the normal beat (N) and ventricular extrasystolic beat (V) for 24 hours. If there is a beat that is erroneously determined, the kind of the beat is corrected. When the electrocardiogram waveform analysis apparatus 100 of the embodiment is used, it is possible to eliminate erroneous determination of beats, and therefore the efficiency of the edition work in the editing center is remarkably improved. Moreover, also the reliability of the electrocardiogram waveform data that, after the edition, are delivered to the hospital is significantly improved.

As illustrated in FIG. 8A, for example, electrocardiogram waveforms (raw waveforms) of a plurality of channels are input from the Holter electrocardiograph to the electrocardiogram waveform analysis apparatus 100 of the embodiment. In FIG. 8A, in order to facilitate the description, electrocardiogram waveforms of two channels or ch 1 and ch 2 are exemplarily illustrated. In the electrocardiogram waveform of ch 1, the amplitudes of the QRS waves are measured to be large, but, in the electrocardiogram waveform of ch 2, those of the QRS waves are measured to be small as compared to the electrocardiogram waveform of ch 1. Even in the case where the electrodes are correctly attached to the subject, the amplitude of an electrocardiogram waveform is sometime measured to be small, as in ch 2. When the electrodes are incompletely attached to the subject, the amplitude of the electrocardiogram waveform of ch 2 is measured to be further smaller.

As illustrated in FIG. 8B, the electrocardiogram waveform analysis apparatus 100 of the embodiment applies a filtering process to portions of the electrocardiogram waveforms other than the QRS wave, to eliminate noises. When the electrocardiogram waveforms (processed waveforms) of the plurality of channels that have undergone the filtering process are compared with the electrocardiogram waveforms of FIG. 8A, it is particularly seen that, in the electrocardiogram waveform of ch 2 in which the amplitude is small as a whole, noises are reduced (the jaggedness of the waveform is reduced). While, viewing the both electrocardiogram waveforms of ch 1 and ch 2, the electrocardiogram waveform analysis apparatus 100 classifies the kinds of beats. When noises remain in one of the electrocardiogram waveforms of ch 1 and ch 2, therefore, an erroneous determination of beats is easily caused by the electrocardiogram waveform of the channel in which noises remain.

The electrocardiogram waveforms of FIG. 8A were analyzed by a conventional electrocardiogram waveform analysis apparatus to which the presently disclosed subject matter is not applied. As a result, as illustrated in FIG. 9A, the electrocardiogram waveforms (raw waveforms) and analysis results of the determined kinds of beats were displayed. In the display of FIG. 9A, the kinds of beats that are enclosed respectively by the ellipses were erroneously determined. From the display, it is seen that the kinds of beats are wrong, and a portion that is not a beat was determined as a beat. In the editing center, therefore, the editor manually deletes or corrects the kinds of beats that are enclosed by the ellipses, while checking the shape of the electrocardiogram waveforms (raw waveforms). The editor performs the correction work on analysis results of 24 hours. The correction work requires a lot of trouble and concentration. When an erroneous determination of beats frequently occurs, therefore, the efficiency of the edition work is significantly lowered. The doctor who diagnoses arrhythmia performs the diagnosis while viewing the analysis results after the correction work. When an erroneous determination of beats remains to be corrected, the doctor has a suspicion about the determination accuracy, and the reliability of the analysis results after the correction work is lowered.

On the other hand, the electrocardiogram waveforms of FIG. 8A were analyzed by the electrocardiogram waveform analysis apparatus 100 of the embodiment to which the presently disclosed subject matter is applied. As a result, as illustrated in FIG. 9B, the electrocardiogram waveforms (raw waveforms) and analysis results of the determined kinds of beats were displayed. From the display, it is seen that no erroneous determination of beats occurs. In the editing center, therefore, the edition work by the editor is not necessary. When the electrocardiogram waveform analysis apparatus 100 of the embodiment is used, therefore, the efficiency of the edition work in the editing center is remarkably improved. The doctor who diagnoses arrhythmia can perform the diagnosis of arrhythmia with confidence in the analysis results in the editing center. Alternatively, the timings of beats (the kinds are not illustrated) may be displayed together with the electrocardiogram waveforms (raw waveforms). Namely, a configuration may be employed where the timings of beats that are detected by the beat detector 140 from the filtered electrocardiogram waveforms, and the electrocardiogram waveforms that have not yet been subjected to the filtering process by the filtering section 130 may be combinedly displayed.

The electrocardiogram waveform analysis apparatus 100 of the embodiment applies a filtering process to the electrocardiogram waveforms such as illustrated in FIG. 8A that are input from the waveform input section 110 (see FIG. 1), and then obtains filtered electrocardiogram waveforms such as illustrated in FIG. 8B. However, the filtered electrocardiogram waveforms are used only in determination of the kinds of beats, and not displayed on the display device, nor printed. The analysis results to be displayed on the display device are finally in the display mode such as illustrated in FIG. 9B. The electrocardiogram waveforms that are to be displayed at this time are the electrocardiogram waveforms (raw waveforms) that are input from the waveform input section 110. The reason why, as described, the kinds of beats are displayed with respect to raw waveforms, and the kinds of beats are not displayed with respect to the electrocardiogram waveforms that have undergone the filtering process is that the person who sees the display has a feeling of strangeness.

Although the electrocardiogram waveform analysis apparatus 100 of the embodiment has been described above, the technical scope of the presently disclosed subject matter is not limited to the description of the embodiment.

For example, the waveform input section 110 is not limited to the configuration where an electrocardiogram waveform that is measured by a patient monitor, a defibrillator, a 12-lead electrocardiograph, or a Holter electrocardiograph is input to the waveform input section. An electrocardiogram waveform that is measured by an apparatus other than the above-described apparatuses may be input to the waveform input section as far as the waveform is an electrocardiogram waveform.

A low-pass filter has been exemplified as the filter. A band-pass filter or a hum filter may be used in place of a low-pass filter.

In place of an apparatus in which the kinds of beats are determined and then displayed as in the embodiment, for example, the presently disclosed subject matter can be applied also to an apparatus in which the kinds of beats are determined and then alarms corresponding to the kinds of beats are output. Specifically, the presently disclosed subject matter can be applied to an apparatus in which, when the ventricular extrasystolic beat (V) is detected, an alarm is output, and, when the normal beat (N) is detected, another alarm is output. Moreover, the presently disclosed subject matter can be applied also to a use in which the kinds of beats are not classified, and simply the heart rate is calculated (beats are not classified, and the number of beats is counted).

In an apparatus to which the presently disclosed subject matter is applied, the apparatus can be used by the operation method that is identical with that which was performed before the presently disclosed subject matter is applied. Moreover, arrhythmia can be determined with an accuracy that is higher than that which is attained before the presently disclosed subject matter is applied.

## Claims

1. An electrocardiogram waveform analysis apparatus (100) comprising:
a waveform input section (110) to which an electrocardiogram waveform is input;
an application range setting section (120) configured to set portions of the electrocardiogram waveform that are other than a QRS wave of the electrocardiogram waveform as an application range of a filter in the input electrocardiogram waveform to define the application range of the filter that excludes the QRS wave of the input electrocardiogram waveform and comprises only one or more portions located outside the QRS wave;
a filtering section (130) configured to apply a filtering process only to the application range of the filter while leaving the QRS wave, the filtering process being configured to reduce noise contained in the filter application range;
a beat detector (140) configured to detect beats from the electrocardiogram waveform that has undergone the filtering process; and
an output section (160) configured to perform an outputting process based on the detected beats.

2. The electrocardiogram waveform analysis apparatus (100) according to claim 1, wherein the electrocardiogram waveform that is input to the waveform input section (110) is an electrocardiogram waveform that extends over a predetermined time period and is measured by a patient monitor, a defibrillator, a 12-lead electrocardiograph, or a Holter electrocardiograph.

3. The electrocardiogram waveform analysis apparatus (100) according to claim 1 or 2, wherein
the application range setting section (120) is configured to set a specific amplitude as a reference, the specific amplitude being in the predetermined time period of the electrocardiogram waveform, the specific amplitude satisfying a predetermined standard, and
the application range setting section (120) is configured to set, as the application range, one or more waveforms having an amplitude that is equal to or smaller than a predetermined ratio of the specific amplitude set as the reference.

4. The electrocardiogram waveform analysis apparatus (100) according to any one of claims 1 to 3, wherein the filtering section (130) is configured to use, as the filter, an analog filter including an RC filter in which a resistor and a capacitor are used or an LC filter in which a coil and a capacitor is used, or a digital filter in which a calculation technique including a moving average method is employed.

5. The electrocardiogram waveform analysis apparatus (100) according to any one of claims 1 to 4, wherein the output section (160) is configured to combine and output the beats detected by the beat detector (140) and the electrocardiogram waveform that is input to the waveform input section and has not yet undergone the filtering process by the filtering section (130).

6. The electrocardiogram waveform analysis apparatus (100) according to any one of claims 1 to 5, wherein the apparatus (100) further comprises a beat analyzer (150) configured to analyze the detected beats, and configured to classify kinds of the beats.

7. The electrocardiogram waveform analysis apparatus (100) according to claim 6, wherein the beat analyzer (150) is configured to classify the kinds of beats by using pattern matching.

8. The electrocardiogram waveform analysis apparatus (100) according to claim 7, wherein the kinds of beats are a plurality of kinds including a normal beat (N) and a ventricular extrasystolic beat (V).

9. The electrocardiogram waveform analysis apparatus (100) according to any one of claims 6 to 8, wherein the output section (160) is configured to output the classified kinds of beats together with the input electrocardiogram waveform.

10. The electrocardiogram waveform analysis apparatus (100) according to any one of claims 1 to 9, wherein the output section (160) is configured to transmit display data that are based on the beats, to an external apparatus.

11. The electrocardiogram waveform analysis apparatus (100) according to any one of claims 1 to 10, wherein the output section (160) is a display device that is a liquid crystal or an organic EL and is configured to perform a display based on the beats, or a printer configured to perform printing based on the beats.

## Patentansprüche

1. Elektrokardiogramm-Wellenform-Analysevorrichtung (100), umfassend:
einen Wellenformeingabeabschnitt (110), in den eine Elektrokardiogramm-Wellenform eingegeben wird;
einen Abschnitt zur Einstellung des Anwendungsbereichs (120), der dazu eingerichtet ist, Abschnitte der Elektrokardiogramm-Wellenform, die von einer QRS-Welle der Elektrokardiogramm-Wellenform verschieden sind, als einen Anwendungsbereich eines Filters in der eingegebenen Elektrokardiogramm-Wellenform festzulegen, um den Anwendungsbereich des Filters zu definieren, der die QRS-Welle der eingegebenen Elektrokardiogramm-Wellenform ausschließt und nur einen oder mehrere außerhalb der QRS-Welle gelegene Abschnitte umfasst;
einen Filterabschnitt (130), der dazu eingerichtet ist, einen Filtervorgang nur auf den Anwendungsbereich des Filters anzuwenden, wobei die QRS-Welle ausgespart wird, wobei der Filtervorgang dazu eingerichtet ist, in dem Anwendungsbereich des Filters enthaltenes Rauschen zu reduzieren;
einen Schlagdetektor (140), der dazu eingerichtet ist, Schläge aus der Elektrokardiogramm-Wellenform zu detektieren, die dem Filtervorgang unterzogen worden ist; und
einen Ausgabeabschnitt (160), der dazu eingerichtet ist, einen Ausgabevorgang auf Grundlage der detektierten Schläge durchzuführen.

2. Elektrokardiogramm-Wellenform-Analysevorrichtung (100) nach Anspruch 1, wobei die Elektrokardiogramm-Wellenform, die in den Wellenformeingabeabschnitt (110) eingegeben wird, eine Elektrokardiogramm-Wellenform ist, die sich über einen vorgegebenen Zeitraum erstreckt und von einem Patientenmonitor, einem Defibrillator, einem 12-Kanal-Elektrokardiographen oder einem Holter-Elektrokardiographen gemessen wird.

3. Elektrokardiogramm-Wellenform-Analysevorrichtung (100) nach Anspruch 1 oder 2, wobei
der Abschnitt zur Einstellung des Anwendungsbereichs (120) dazu eingerichtet ist, eine spezifische Amplitude als eine Referenz festzulegen, wobei die spezifische Amplitude in dem vorgegebenen Zeitraum der Elektrokardiogramm-Wellenform liegt, wobei die spezifische Amplitude einen vorgegebenen Standard erfüllt, und
der Abschnitt zur Einstellung des Anwendungsbereichs (120) dazu eingerichtet ist, als den Anwendungsbereich eine oder mehrere Wellenformen festzulegen, die eine Amplitude aufweisen, die gleich oder kleiner als ein vorgegebenes Verhältnis der als Referenz festgelegten spezifischen Amplitude ist.

4. Elektrokardiogramm-Wellenform-Analysevorrichtung (100) nach einem der Ansprüche 1 bis 3, wobei der Filterabschnitt (130) dazu eingerichtet ist, als den Filter einen Analogfilter zu verwenden, der einen RC-Filter, bei dem ein Widerstand und ein Kondensator verwendet werden, oder einen LC-Filter, bei dem eine Spule und ein Kondensator verwendet wird, umfasst, oder einen Digitalfilter, bei dem eine Berechnungstechnik einschließlich eines Verfahrens des gleitenden Mittelwerts eingesetzt wird.

5. Elektrokardiogramm-Wellenform-Analysevorrichtung (100) nach einem der Ansprüche 1 bis 4, wobei der Ausgabeabschnitt (160) dazu eingerichtet ist, die von dem Schlagdetektor (140) detektierten Schläge und die Elektrokardiogramm-Wellenform, die in den Wellenformeingabeabschnitt eingegeben wird und noch nicht dem Filtervorgang durch den Filterabschnitt (130) unterzogen worden ist, zu kombinieren und auszugeben.

6. Elektrokardiogramm-Wellenform-Analysevorrichtung (100) nach einem der Ansprüche 1 bis 5, wobei die Vorrichtung (100) ferner einen Schlaganalysator (150) umfasst, der dazu eingerichtet ist, die detektierten Schläge zu analysieren, und dazu eingerichtet ist, Arten der Schläge zu klassifizieren.

7. Elektrokardiogramm-Wellenform-Analysevorrichtung (100) nach Anspruch 6, wobei der Schlaganalysator (150) dazu eingerichtet ist, die Schlagarten unter Verwendung eines Musterabgleichs zu klassifizieren.

8. Elektrokardiogramm-Wellenform-Analysevorrichtung (100) nach Anspruch 7, wobei die Schlagarten eine Mehrzahl von Arten sind, die einen Normalschlag (N) und einen ventrikulären extrasystolischen Schlag (V) umfassen.

9. Elektrokardiogramm-Wellenform-Analysevorrichtung (100) nach einem der Ansprüche 6 bis 8, wobei der Ausgabeabschnitt (160) dazu eingerichtet ist, die klassifizierten Schlagarten zusammen mit der eingegebenen Elektrokardiogramm-Wellenform auszugeben.

10. Elektrokardiogramm-Wellenform-Analysevorrichtung (100) nach einem der Ansprüche 1 bis 9, wobei der Ausgabeabschnitt (160) dazu eingerichtet ist, Anzeigedaten, die auf den Schlägen basieren, an eine externe Vorrichtung zu übertragen.

11. Elektrokardiogramm-Wellenform-Analysevorrichtung (100) nach einem der Ansprüche 1 bis 10, wobei der Ausgabeabschnitt (160) eine Anzeigevorrichtung ist, die ein Flüssigkristall oder eine organische EL ist und dazu eingerichtet ist, eine Anzeige auf Grundlage der Schläge durchzuführen, oder ein Drucker, der dazu eingerichtet ist, einen Druckvorgang auf Grundlage der Schläge durchzuführen.

## Revendications

1. Appareil d'analyse de forme d'onde d'électrocardiogramme (100) comprenant :
une section d'entrée de forme d'onde (110) dans laquelle une forme d'onde d'électrocardiogramme est entrée ;
une section de réglage de la plage d'application (120) configurée pour définir des portions de la forme d'onde d'électrocardiogramme qui sont autres qu'une onde QRS de la forme d'onde d'électrocardiogramme comme une plage d'application d'un filtre dans la forme d'onde d'électrocardiogramme d'entrée, afin de définir la plage d'application du filtre qui exclut l'onde QRS de la forme d'onde d'électrocardiogramme d'entrée et comprend uniquement une ou plusieurs portions situées à l'extérieur de l'onde QRS ;
une section de filtrage (130) configurée pour appliquer un processus de filtrage uniquement à la plage d'application du filtre tout en laissant de côté l'onde QRS, le processus de filtrage étant configuré pour réduire le bruit contenu dans la plage d'application du filtre ;
un détecteur de battements (140) configuré pour détecter des battements à partir de la forme d'onde d'électrocardiogramme qui a subi le processus de filtrage ; et
une section de sortie (160) configurée pour effectuer un processus de sortie sur la base des battements détectés.

2. Appareil d'analyse de forme d'onde d'électrocardiogramme (100) selon la revendication 1, dans lequel la forme d'onde d'électrocardiogramme qui est entrée dans la section d'entrée de forme d'onde (110) est une forme d'onde d'électrocardiogramme qui s'étend sur une période de temps prédéterminée et est mesurée par un moniteur de patient, un défibrillateur, un électrocardiographe à 12 dérivations ou un électrocardiographe Holter.

3. Appareil d'analyse de forme d'onde d'électrocardiogramme (100) selon la revendication 1 ou 2, dans lequel
la section de réglage de la plage d'application (120) est configurée pour définir une amplitude spécifique comme référence, l'amplitude spécifique se trouvant dans la période de temps prédéterminée de la forme d'onde d'électrocardiogramme, l'amplitude spécifique satisfaisant à un critère prédéterminé, et
la section de réglage de la plage d'application (120) est configurée pour définir, comme plage d'application, une ou plusieurs formes d'onde ayant une amplitude qui est égale ou inférieure à un rapport prédéterminé de l'amplitude spécifique définie comme référence.

4. Appareil d'analyse de forme d'onde d'électrocardiogramme (100) selon l'une quelconque des revendications 1 à 3, dans lequel la section de filtrage (130) est configurée pour utiliser, comme filtre, un filtre analogique comprenant un filtre RC dans lequel une résistance et un condensateur sont utilisés ou un filtre LC dans lequel une bobine et un condensateur est utilisé, ou un filtre numérique dans lequel une technique de calcul comprenant une méthode de moyenne mobile est employée.

5. Appareil d'analyse de forme d'onde d'électrocardiogramme (100) selon l'une quelconque des revendications 1 à 4, dans lequel la section de sortie (160) est configurée pour combiner et délivrer les battements détectés par le détecteur de battements (140) et la forme d'onde d'électrocardiogramme qui est entrée dans la section d'entrée de forme d'onde et qui n'a pas encore subi le processus de filtrage par la section de filtrage (130).

6. Appareil d'analyse de forme d'onde d'électrocardiogramme (100) selon l'une quelconque des revendications 1 à 5, dans lequel l'appareil (100) comprend en outre un analyseur de battements (150) configuré pour analyser les battements détectés, et configuré pour classifier les types de battements.

7. Appareil d'analyse de forme d'onde d'électrocardiogramme (100) selon la revendication 6, dans lequel l'analyseur de battements (150) est configuré pour classifier les types de battements en utilisant une mise en correspondance de motifs.

8. Appareil d'analyse de forme d'onde d'électrocardiogramme (100) selon la revendication 7, dans lequel les types de battements sont une pluralité de types comprenant un battement normal (N) et un battement extrasystolique ventriculaire (V).

9. Appareil d'analyse de forme d'onde d'électrocardiogramme (100) selon l'une quelconque des revendications 6 à 8, dans lequel la section de sortie (160) est configurée pour délivrer les types de battements classifiés conjointement avec la forme d'onde d'électrocardiogramme d'entrée.

10. Appareil d'analyse de forme d'onde d'électrocardiogramme (100) selon l'une quelconque des revendications 1 à 9, dans lequel la section de sortie (160) est configurée pour transmettre à un appareil externe des données d'affichage qui sont basées sur les battements.

11. Appareil d'analyse de forme d'onde d'électrocardiogramme (100) selon l'une quelconque des revendications 1 à 10, dans lequel la section de sortie (160) est un dispositif d'affichage qui est un cristal liquide ou une EL organique et est configuré pour effectuer un affichage sur la base des battements, ou une imprimante configurée pour effectuer une impression sur la base des battements.
